# EUROPEAN PATENT APPLICATION

(11) **EP 3 922 711 A1**
(43) Date of publication of application: **15.12.2021**
(21) Application number: 20752716.9
(22) Date of filing: 04.02.2020
(51) Int. Cl.: C12M 3/00, C12M 3/06, C12M 3/08, B03C 1/00

(54) **SAMPLE TREATING DEVICE**

(30) Priority: 05.02.2019 JP 2019018830
(71) Applicant: Universal Bio Research Co., Ltd., Matsudo-shi Chiba 271-0064 (JP)
(72) Inventor: TAJIMA Hideji, Matsudo-shi, Chiba 271-0064 (JP)
(74) Representative: J A Kemp LLP
(86) International application number: PCT/JP2020/004054
(87) International publication number: WO 2020/162431

(57) **Abstract**

This sample treating device 1000 is provided with: a treating stage 110 for treating a sample by mixing the sample and a plurality of solutions; a first motor 812 for rotating the treating stage 110 around a first rotation shaft 810; a second motor for rotating the treating stage 110 around a second shaft 820; and a control unit for controlling the rotations of the treating stage driven by the first rotary motor 812 and the second motor. The control unit combines the operation of rotating the treating stage 110 around the first rotation shaft 810 and the operation of rotating the treating stage 110 around the second rotation shaft, thereby treating the sample.

## Description

### TECHNICAL FIELD

The present invention relates to a sample treating device which can treat a sample by imparting difference in elevation to a sample treating stage.

### BACKGROUND ART

Separation of a component of interest (biological substance) from blood is performed in a centrifuge, where the blood and the separated component are transferred among containers by application of pressure. For example, the blood component collection device of Patent document 1 uses a centrifuge to separate a component from blood, and the component separated from the blood is transferred to a bag via a tube by a fluid delivery pump ([0029], [0030]).

Meanwhile, the applicant of the present application proposes to conduct cell culture and fractionation of the cultured cells by utilizing fall of the solution upon cell culture in the culture system of Patent document 2 or the culture device of Patent document 3.

### PRIOR ART DOCUMENTS

### Patent documents

Patent document 1: Japanese Unexamined Patent Application Publication No. 2006-109978
Patent document 2: International Patent Application Publication WO2015/037468
Patent document 3: International Patent Application Publication WO2015/005299

### SUMMARY OF INVENTION

### Problem to be solved by invention

In the blood component collection device of Patent document 1, a centrifuge is used for separating a component contained in the blood, which not only requires the device to be made on a large scale but also applies stress to the blood upon centrifugation and may have an adverse effect to the delicate biological substance contained in the blood (for example, destruction of the substance). Since the culture system of Patent document 2 and the culture device of Patent document 3 are provided with only one rotation shaft for allowing the solution containing the biological substance to fall, they may have difficulty in performing multiple treatments.

Thus, the present invention has an objective of providing a sample treating device which can treat a sample containing a biological substance under relatively low stress conditions. Alternatively, the present invention has an objective of providing a sample treating device which can perform multiple treatments upon treating a sample by transferring a solution containing a biological substance by the fall thereof.

### Means for solving problem

The aspects of the present invention are as follows.

### (Aspect 1)

A sample treating device for treating a solution containing a sample to obtain a solution containing a biological substance of interest from the sample, the device comprising:
a treating stage for performing the treatment of the sample by mixing the solution containing the sample with a plurality of solutions;
a first rotating device for rotating the treating stage about a first rotation shaft;
a second rotating device for rotating the treating stage about a second rotation shaft which is different from the first rotation shaft; and
a controller for controlling rotations by the first rotating device and the second rotating device,
wherein the controller tilts the treating stage in multiple directions by using the first rotating device and the second rotating device for carrying out the treatment, and the controller utilizes these tilts in multiple directions to transfer the solution containing the sample and/or the solution containing the biological substance to perform the treatment.

### (Aspect 2)

The sample treating device according to Aspect 1, wherein the controller directs an operation of rotating the treating stage about the first rotation shaft and an operation of rotating the treating stage about the second rotation shaft for treating the sample.

### (Aspect 3)

The sample treating device according to either one of Aspects 1 and 2, comprising:
a frame for rotatably holding the treating stage; and
a support for rotatably supporting the frame.

### (Aspect 4)

The sample treating device according to Aspect 2, wherein the frame is rotatable in one piece with the first rotation shaft.

### (Aspect 5)

The sample treating device according to either one of Aspects 3 and 4, wherein the first rotating device is provided on the support.

### (Aspect 6)

The sample treating device according to any one of Aspects 1-5, wherein the treating stage is rotatable in one piece with the second rotation shaft.

### (Aspect 7)

The sample treating device according to Aspect 6, wherein the second rotating device is provided on the frame.

### (Aspect 8)

The sample treating device according to any one of Aspects 1-7, comprising a solution supply section which allows the solution containing the sample and the plurality of solutions to transfer to the treating stage by the fall thereof.

### (Aspect 9)

The sample treating device according to any one of Aspects 1-8, wherein the treating stage is provided with a biological substance separating unit for separating the biological substance from the sample by using magnetic particles.

### (Aspect 10)

The sample treating device according to Aspect 9, comprising a magnet plate for adsorbing the magnetic particles in the biological substance separating unit.

### (Aspect 11)

The sample treating device according to Aspect 10, comprising a magnetic force controller for controlling the magnetic force of the magnet plate.

### (Aspect 12)

The sample treating device according to either one of Aspects 10 and 11, wherein the controller tilts the treating stage using the first rotating device and/or the second rotating device while the biological substance having the magnetic particles attached thereto is adsorbed in the biological substance separating unit by the magnetic force of the magnet plate, thereby separating the biological substance having the magnetic particles attached thereto from the sample.

### (Aspect 13)

The sample treating device according to Aspect 12, wherein the controller supplies a wash solution to the biological substance separating unit while the biological substance having the magnetic particles attached thereto is adsorbed in the biological substance separating unit, thereby separating the magnetic particles from the biological substance.

### (Aspect 14)

The sample treating device according to Aspect 13, wherein the treating stage is provided with a filter unit for filtering the biological substance from the solution containing the biological substance separated from the magnetic particles.

### (Aspect 15)

The sample treating device according to Aspect 14, comprising a solution pressurizer for applying a pressure to the solution passing through the filter unit to promote the filtration.

### (Aspect 16)

The sample treating device according to Aspect 15, wherein the solution pressurizer is a bellows or a gas supply unit.

### (Aspect 17)

The sample treating device according to any one of Aspects 14-16, wherein the controller tilts the treating stage so that the solution containing the biological substance is transferred from the biological substance separating unit to the filter unit by the fall thereof.

### (Aspect 18)

The sample treating device according to Aspect 17, wherein the filter unit is provided with a first chamber, a filter section connected to the first chamber and a second chamber connected to the filter section, wherein the first chamber, the filter section and the second chamber are arranged along the first rotation shaft.

### (Aspect 19)

The sample treating device according to Aspect 18, wherein the controller tilts the treating stage using the second rotating device so that the solution containing the biological substance is transferred to the first chamber by the fall thereof, the biological substance is filtered from the solution at the filter section, and the solution is disposed via the second chamber.

### (Aspect 20)

The sample treating device according to Aspect 19, wherein the controller supplies a wash solution to the first chamber while the treating stage is positioned to be in the tilted state to wash the biological substance filtered at the filter section.

### (Aspect 21)

The sample treating device according to any one of Aspects 14-20, wherein the treating stage is provided with a manipulation unit for manipulating the biological substance filtered by the filter unit.

### (Aspect 22)

The sample treating device according to Aspect 21, wherein the filter unit and the manipulation unit are arranged along the second rotation shaft, and the controller tilts the treating stage using the first rotating device so that the solution containing the biological substance is transferred from the filter unit to the manipulation unit by the fall thereof.

### (Aspect 23)

The sample treating device according to Aspect 22, wherein the biological substance is a cell, the manipulation unit is a culture unit for culturing the cell, and the stage is provided with a temperature regulating unit for regulating the temperature of the culture unit.

### (Aspect 24)

The sample treating device according to Aspect 23, wherein, for culturing the cell, the controller directs an operation of swinging the treating stage about the first rotation shaft and/or directs an operation of swinging the treating stage about the second rotation shaft, thereby culturing the cells.

### (Aspect 25)

The sample treating device according to any one of Aspects 1-24, comprising, beneath the treating stage, a biological substance storage section for storing the solution containing the biological substance.

### (Aspect 26)

The sample treating device according to Aspect 25, wherein the biological substance storage section is provided with a cooler for cooling the solution containing the biological substance.

### (Aspect 27)

The sample treating device according to either one of Aspects 25 and 26, wherein the biological substance storage section is provided with a measurement mechanism for measuring the biological substance.

### (Aspect 28)

A method for treating a sample by using the sample treating device according to any one of Aspects 1-27, the method comprising the steps of:
tilting the treating stage in a first direction using the first rotating device; and
tilting the treating stage in a second direction using the second rotating device.

### EFFECT OF THE INVENTION

A sample treating device of the present invention is capable of treating a sample containing a biological substance under relatively low stress conditions.

### BREIF DESCRIPTION OF DRAWINGS

[Figure 1] A perspective view showing a sample treating device according to an embodiment of the present invention.
[Figure 2] A perspective view of the sample treating device shown in Figure 1, where the housing is omitted in the drawing.
[Figure 3] A cross-sectional side view of the sample treating device shown in Figure 1.
[Figure 4] A cross-sectional view of the sample treating device shown in Figure 3 taken along line A-A.
[Figure 5] A cross-sectional view of the sample treating device shown in Figure 3 taken along line B-B.
[Figure 6] A perspective view showing a state upon rotating the treatment section of Figure 2.
[Figure 7] A perspective view showing a state upon rotating the treating stage of Figure 2.

### MODES FOR CARRYING OUT INVENTION

A sample treating device according to an embodiment of the present invention will be described with reference to the drawings. Terms used for the embodiment of the present invention are defined as follows. The term "sample" includes any substance obtained from an organism, for example, whole blood (blood), cerebrospinal fluid, pus, an aspirate, urine, feces, a part of tissue or the like. The term "treatment" includes mixing, stirring, extraction, separation, purification, filtration, culture, cell differentiation and the like. The term "biological substance" includes any substance contained in a sample, for example, a protein, a cell, nucleic acids (RNA, DNA), a lipid, an exosome or the like. Here, the same components are denoted by the same reference numerals throughout the figures and the descriptions thereof are properly omitted.

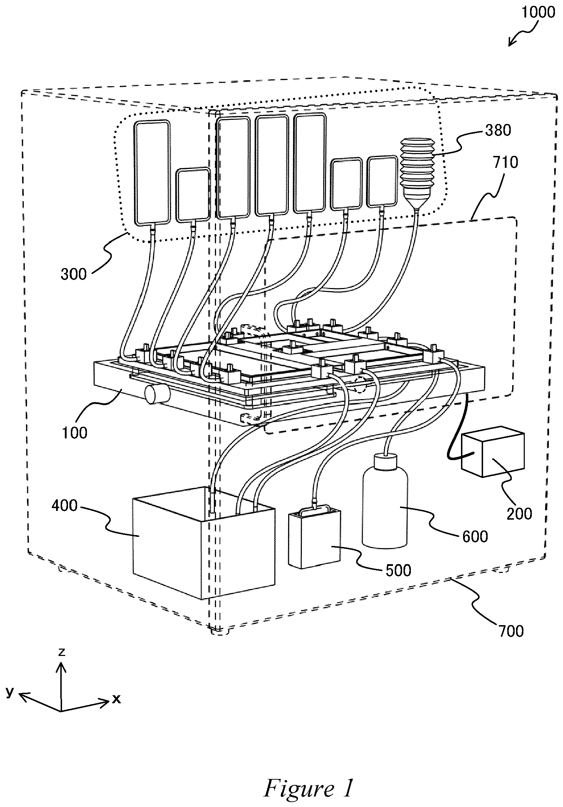

Figure 1 shows a perspective view of a sample treating device 1000 according to an embodiment of the present invention. The sample treating device 1000 comprises at least a treatment section 100 for treating a sample, a controller 200 for controlling operations of the treatment section 100, and a solution supply section 300 provided above the treatment section 100. Furthermore, the sample treating device 1000 preferably comprises any one or more of the followings: a liquid waste tank 400 provided beneath the treatment section 100; a biological substance storage section 500 provided beneath the treatment section 100; a CO₂ gas supply unit (CO₂ gas cylinder) 600 provided beneath the treatment section 100; and a housing 700 for accommodating at least the treatment section 100.

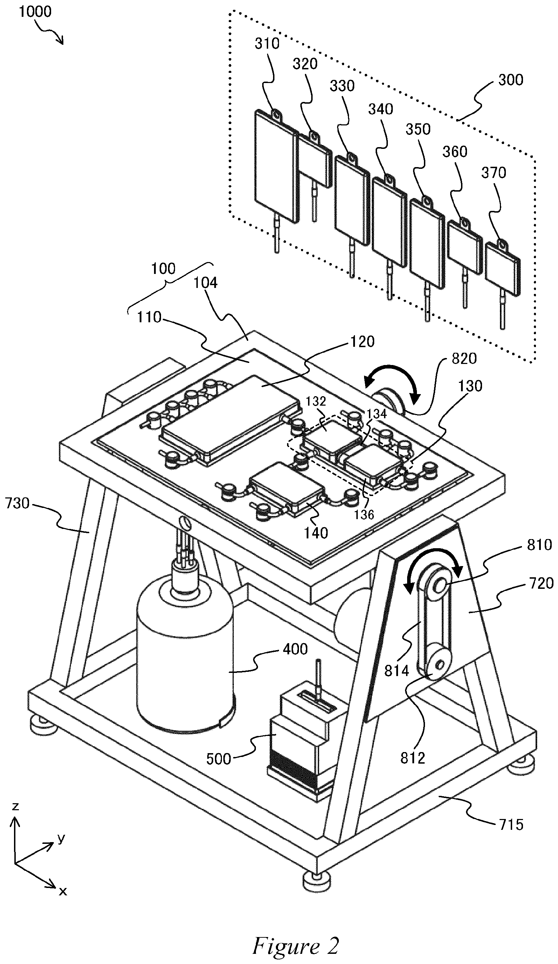

As will be described later, the treatment section 100 comprises a generally flat treating stage 110 and two rotation shafts 810 and 820 (Figure 2). In Figure 2, the flat treating stage 110 is shown in a substantially horizontal state. The treatment section 100 performs treatments by tilting each of units (bag or container) mounted on and secured to the substantially horizontal treating stage in multiple directions using the two rotation shafts 810 and 820. The units of the treatment section 100 are connected to bags or the like of the solution supply section 300 arranged above the treatment section 100. Each of the tubes is provided with a valve whose opening/closing is controlled by the controller 200. Preferably, a pinch valve is used as the valve, where the pinch valve can close the tube by pinching the flexible tube from outside. Preferably, the tube is a silicone tube.

In addition to the treatment section 100, the housing 700 can accommodate any one or more of the followings: a controller (for example, personal computer) 200; a solution supply section 300; a liquid waste tank 400; a biological substance storage section 500; and a CO₂ gas supply unit 600. The housing 700 is provided with a window 710 made of glass or a transparent resin so that the states of the treatment section 100 and the solution supply section 300 can be visually observed through the window 710.

Figure 2 is a upper perspective view of the sample treating device 1000 shown in Figure 1 with the housing 700 being omitted. The solution supply section 300 preferably includes any one or more of the followings: a whole blood-containing bag (sample container) 310; a magnetic bead solution bag (magnetic bead solution container) 320; a separating agent solution bag (reagent solution container) 330; a first wash solution bag (first wash solution container) 340; a second wash solution bag (second wash solution container) 350; a culture reagent solution bag (culture reagent solution container) 360; and a culture solution bag (culture solution container) 370. When a solution in a bag (flexible bag) of the solution supply section 300 is allowed to transfer by the fall thereof, an air venting needle (aeration needle) is preferably stuck into the bag to promote the solution in the bag to fall. The vent of the air venting needle is provided with a filter. When a less deformable container is used instead of a bag in the solution supply section 300, the container is preferably provided with a vent with a lid. When a solution is allowed to transfer from the container by the fall thereof, the lid is preferably loosened to allow ventilation via the vent to promote the solution inside the container to fall. The vent of the container is provided with a filter.

Although not shown in Figure 2, the solution supply section 300 is preferably provided with a bellows (solution pressurizer) 380 shown in Figure 1. The bellows 380 is preferably compressed (pressed) by an actuator (not shown) to apply a pressure to a filter unit 130 described below to transfer the solution. Alternatively, a CO₂ gas supply unit 600 may favorably be used as a solution pressurizer instead of the bellows. Each of the bags 310-370 and/or the bellows 380 is preferably made of a flexible resin and provided with a hang tab on the top. This tab can be hanged on a hook or the like provided inside the housing 700 so that the bags 310-370 and/or the bellows 380 can be kept at positions higher than the treatment section 100 within the housing 700. In Figures 2-7, tubes connecting the bags and the bellows 380 of the solution supply section 300 with the treatment section 100 are omitted.

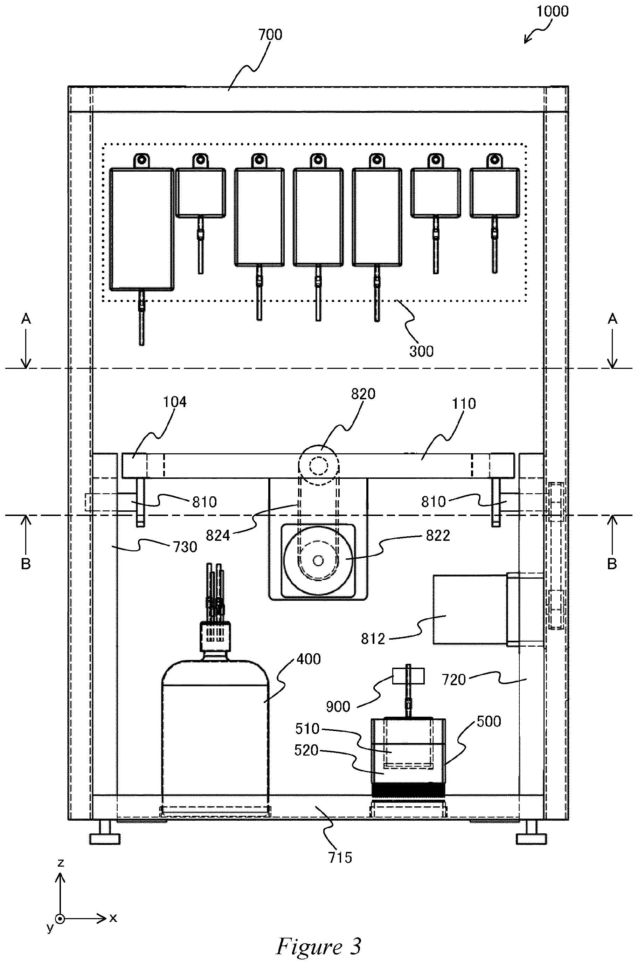

As shown in Figure 3, the biological substance storage section 500 is preferably provided with either or both of a storage bag (storage container) 510 and a cooler (e.g., Peltier device) 520 for cooling the storage bag 510. Furthermore, the tube connecting the biological substance storage section 500 and the treatment section 100 (culture unit 140 described below) is preferably provided with a magnetic force unit 900. A minute amount of magnetic particles may be remaining in the solution containing the biological substance which is transferred from the treatment section 100 to the biological substance storage section 500 by the fall thereof. Accordingly, the magnetic force unit 900 exerts a magnetic force on the solution containing the biological substance that has been treated at the treatment section 100. If a minute amount of magnetic particles is contained in the solution, these magnetic particles can be adsorbed by the magnetic force.

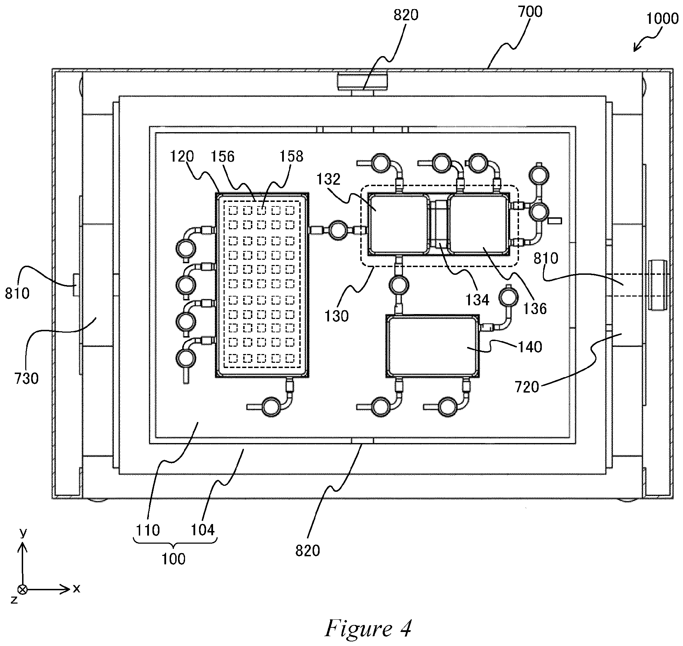

As shown in Figures 2 and 4, the treatment section 100 comprises a frame 104, a treating stage 110 rotatably held by the frame 104, a cell separating unit (biological substance separating unit) 120 detachably disposed on the treating stage 110, a filter unit (filtering section) 130 detachably disposed on the treating stage 110, and a culture unit (manipulation unit) 140 detachably disposed on the treating stage 110. Preferably, a whole blood-containing bag 310, a magnetic bead solution bag 320, a separating agent solution bag 330 and a first a wash solution bag 340 are connected to the cell separating unit 120 via tubes so that the solution in each bag can flow into the cell separating unit 120. The treating stage 110 is a generally flat plate member. In Figures 2-4, the flat surface of the treating stage 110 is shown in a substantially horizontal standby state.

As shown in Figure 4, the filter unit 130 is preferably disposed adjacent to the cell separating unit 120 in the x-direction. The filter unit 130 comprises a first chamber 132 connected to the cell separating unit 120 in the x-direction via a tube, a filter section 134 connected to the first chamber 132 in the x-direction, and a second chamber 136 connected to the filter section 134 in the x-direction. The first chamber 132, the filter chamber 134 and the second chamber 136 are disposed along the x-direction (the first rotation shaft 810). To the first chamber 132, a second wash solution bag 350 is connected via a tube. To the second chamber 136, a culture reagent solution bag 360, a culture solution bag 370 and a bellows 380 are connected via tubes. The filter section 134 is provided with a filter or a dialysis membrane for filtering the biological substance.

The culture unit 140 is disposed adjacent to the first chamber 132 of the filter unit 130 in the y-direction, and connected to the first chamber 132 via a tube. To the culture unit 140, the liquid waste tank 400 for receiving the liquid waste from each unit, the biological substance storage section 500 for storing the biological substance obtained by the treatment, and the CO₂ gas supply unit 600 are connected via tubes.

As shown in Figures 2 and 3, the sample treating device 1000 is preferably provided with a pair of supports 720 and 730 for supporting the treatment section 100 on the bottom frame 715 of the housing 700. The sample treating device 1000 is provided with a first rotation shaft 810 extending in the x-direction, a first motor (first driver) 812 for rotating the first rotation shaft 810, and a drive belt 814 for transmitting rotation of the first motor 812 to the first rotation shaft 810. Both ends of the first rotation shaft 810 are rotatably supported by the pair of supports 720 and 730. The first motor 812 is fixed to the support 720. The treatment section 100 is fixed to the first rotation shaft 810. Accordingly, the treatment section 100 rotates integrally with the first rotation shaft 810. In Figure 3, the units and members provided on the upper and bottom surfaces of the treating stage 110 are not shown.

As shown in Figures 2 and 3, the sample treating device 1000 is provided with a second rotation shaft 820 extending in the y-direction, a second motor (second driver) 822 for rotating the second rotation shaft 820, and a drive belt 824 for transmitting rotation of the second motor 822 to the second rotation shaft 820. Both ends of the second rotation shaft 820 are rotatably supported by the frame 104. The second motor 822 is fixed to the frame 140. The treating stage 110 is fixed to the second rotation shaft 820. Accordingly, the treating stage 110 rotates integrally with the second rotation shaft 820. Preferably, the axial direction of the first rotation shaft 810 is arranged in a direction different from the axial direction of the second rotation shaft 820 or in a direction intersecting the axial direction of the second rotation shaft 820. More preferably, the axial direction of the first rotation shaft 810 is arranged in a direction that makes an angle of about 90 degrees with the axial direction of the second rotation shaft 820.

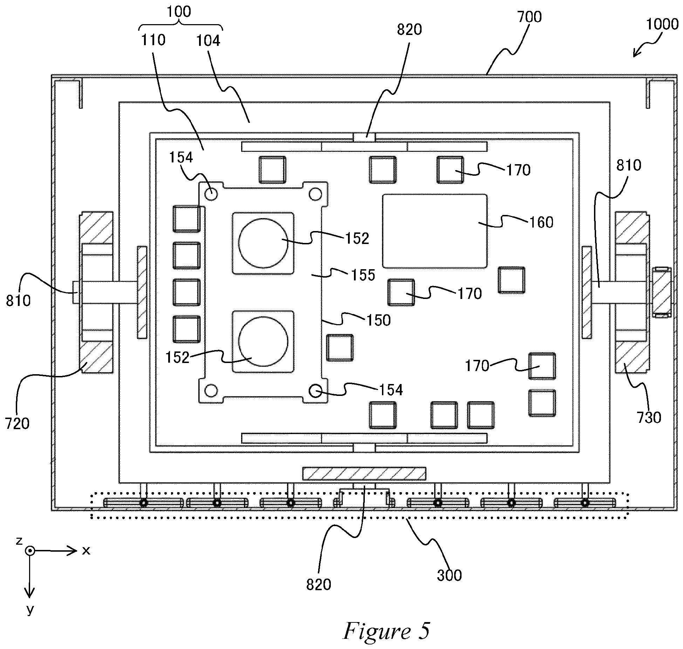

As shown in Figure 5, the bottom surface of the treatment section 100 is preferably provided with any one or more of the followings: a magnetic force-exerting unit 150 for exerting a magnetic force inside the cell separating unit 120; a temperature regulating unit 160 for regulating the temperature inside the culture unit 140 to a temperature suitable for a treatment such as culture; and a valve actuator (solenoid) 170 for actuating each of the valves that open and close the tubes. More preferably, the magnetic force-exerting unit 150 is provided with a plurality of lifting shafts 154 extending downward (in the z-direction) from the bottom surface of the treatment section 100, a magnet plate (permanent magnet) 155 that moves toward and away (in the z-direction) from the bottom surface of the cell separating unit 120 while facing said bottom surface, a magnet arrangement area 156 provided within the magnet plate 155 (Figure 4), and a third motor (third driver) 152 for driving the magnet plate 155. The operation of the third motor (third driver) 152 is controlled by the controller 200. The magnet plate 155 and the third motor 152 form a magnetic force-controlling mechanism. Alternatively, the magnetic force-exerting unit 150 can be composed of an electromagnet. In this case, the magnetic force of the electromagnet is controlled by the controller 200.

As shown in Figure 4, a plurality of permanent magnets 158 are evenly arranged in the magnet arrangement area 156. The permanent magnets can preferably be arranged such that adjacent permanent magnets have different polarities. Accordingly, the biological substance (e.g., cells) having the magnetic particles attached thereto moves to the place where the plurality of permanent magnets 158 exist in the cell separating unit 120. Here, the magnetic beads are coupled to an antibody for the specific biological substance contained in the sample. Such specific biological substance may be, for example, a protein, a cell, nucleic acids (RNA or DNA), an exosome or the like.

In the sample treating device according to the embodiment of the present invention, the bags of the solution supply section 300, the units on the treating stage 100 and the tubes are preferably sterilized in advance, and they can be exchanged or disposed after the sample treatment is completed. In the sample treating device according to the embodiment of the present invention, each unit arranged on the upper surface of the treating stage 110 of the treatment section 100 may have a sealed structure and the sample treating steps can be automated. By doing so, the treatment can be carried out without a manual operation, which can reduce the possibility of contamination.

The sample treating device according to the embodiment of the present invention can be used to automatically take out cells of interest including a cancer cell or the like (for example, erythrocytes, leukemia cells or circulating tumor cells (CTC)), a human leukocyte antigen (HLA), exosomes or nucleic acids such as microRNA from a large amount (several hundred ml) of sample such as whole blood.

In the sample treating device according to the embodiment of the present invention, the culture unit 140 is preferably used as a mixing or manipulation unit for mixing with a biological substance-detecting reagent (labeled antibody) or for manipulating therewith, and the biological substance storage section 500 can be provided with a measurement mechanism for measuring the biological substance bound to the labeled antibody. In this case, the measurement mechanism of the biological substance storage section 500 can be used for detecting and quantifying the biological substance and the like. This measurement mechanism may be composed of, for example, a light-transmitting optical fiber for transmitting excitation light from a light source to the storage bag 510, a light-collecting optical fiber for collecting fluorescent light emitted from the labeled antibody irradiated with the light, and a detection element for detecting fluorescent light from the light-collecting optical fiber.

In the sample treating device according to the embodiment of the present invention, the treatment time spent for obtaining a biological substance of interest from a sample can be relatively long, for example, preferably 12 hours to about 36 hours, more preferably 20 hours to 28 hours, and still more preferably about 24 hours so that no stress is applied to the delicate biological substance.

If the CO₂ gas supply unit 600 is used as a solution pressurizer in the sample treating device according to the embodiment of the present invention, the CO₂ gas supply unit 600 can be connected to a predetermined unit to apply a relatively low pressure to said unit so as to promote delivery of the solution in the tilted predetermined unit. The predetermined unit may be, for example, the cell separating unit 120, the filter unit 130 or the culture unit 140.

In the sample treating device according to the embodiment of the present invention, the angle of the treatment section 100 (frame 104) tilted by the first rotation shaft 810 and/or the angle of the treating stage 110 tilted by the second rotation shaft 820 is preferably in a range of 0-180 degrees and more preferably in a range of 0-90 degrees, and can be varied according to the specific treatment.

### EXAMPLES

A treatment operation using the sample treating device 1000 of this embodiment will be described, in which cells of interest such as immune cells (biological substance) are separated (isolated) from human whole blood (sample), and highly active cells of interest such as more active immune cells are obtained from the separated cells of interest by culture or the like.

First, a step of supplying solutions to the cell separating unit 120 is performed. When a solution is transferred by the fall thereof, the valve provided on each of the tubes is opened in response to the command from the controller 200. The whole blood (for example, 200 ml or 400 ml) in the whole blood-containing bag 310 is transferred to the cell separating unit 120 by the fall thereof via the tube. The magnetic bead solution (for example, 50 ml) in the magnetic bead solution bag 320 is transferred to the cell separating unit 120 by the fall thereof via the tube. The separating agent solution (for example, 200 ml) in the separating agent solution bag 330 is transferred to the cell separating unit 120 by the fall thereof via the tube.

The controller 200 directs a mixing step for mixing the solutions supplied to the cell separating unit 120. In the mixing step, the controller 200 drives the first motor 812 for a predetermined period of time to rotate the first rotation shaft 810 clockwise and anti-clockwise alternately within a predetermined angle range so as to swing the treatment section 100. The predetermined angle range may be, for example a range of ±15 to ±30 degrees with respect to the horizontal state.

By swinging the treatment section 100, the solution in the cell separating unit 120 provided in the treatment section 100 can be shaken or stirred. The controller 200 may also drive the second motor 822 instead of the first motor 812 for a predetermined period of time to rotate the second rotation shaft 820 clockwise and anti-clockwise alternately within a predetermined angle range so as to swing the treating stage 110. Furthermore, the first motor 812 and the second motor 822 can be driven simultaneously to perform complicated shaking operations.

At the end of the mixing step, the controller 200 performs a cell adsorbing step. After turning off the first motor 812 and the second motor 822 when the frame 104 and the treating stage 110 of the treatment section 100 are in the horizontal state, the controller 200 elevates the magnet plate 155 to the adsorbing position where the magnet plate 155 is positioned in the vicinity of or to make contact with the bottom surface of the cell separating unit 120. At the adsorbing position, the plurality of permanent magnets 158 in the magnet arrangement area 156 exert magnetic force on the magnetic beads in the cell separating unit 120. Since the cells of interest in the solution in the cell separating unit 120 are attached to magnetic beads coupled to an antibody for the cell of interest, when the magnet plate 155 is transferred to the adsorbing position, the cells of interest contained in the solution move towards and adsorbed by the plurality of magnets 158 shown in Figure 4.

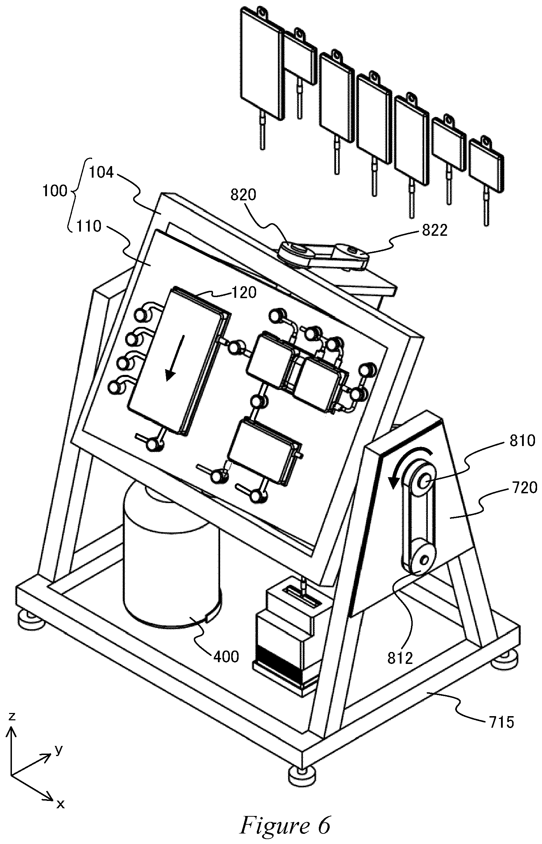

Following the cell adsorbing step, the controller 200 performs a liquid waste disposal step. While the cells of interest are adsorbed to the plurality of magnets, the controller 200 tilts the treatment section 100 at a predetermined angle as shown in Figure 6. The predetermined angle may be, for example, 60-90 degrees with respect to the horizontal state. While the treatment section 100 is tilted, the valve of the tube connecting the cell separating unit 120 and the liquid waste tank 400 is opened for a predetermined period of time so that the unnecessary solution other than the cells having the magnetic particles attached thereto is transferred to the liquid waste tank 400 by the fall thereof via the tube. After a predetermined period of time, the controller 200 closes the valve of the tube connecting the cell separating unit 120 and the liquid waste tank 400, and moves the treatment section 100 to the horizontal position, thereby completing the liquid waste disposal step.

At the end of the liquid waste disposal step, the controller 200 performs a wash solution supplying step. In the wash solution supplying step, the controller 200 opens the valve of the tube connecting the wash solution bag 340 and the cell separating unit 120 so that the wash solution in the wash solution bag 340 is transferred to the cell separating unit 120 by the fall thereof.

After the wash solution supplying step is completed, the controller 200 performs a washing step. In the washing step, the controller 200 lowers the magnet plate 155 and swings the cell separating unit 120 in the same motion as in the mixing step while the magnetic force is not transmitted to the cell separating unit 120, thereby mixing the cells of interest having the magnetic beads attached thereto and the wash solution for a predetermined period of time in the cell separating unit 120. Once the cells of interest and the wash solution are sufficiently mixed, the magnetic beads are separated from the cells of interest, thereby completing the washing step.

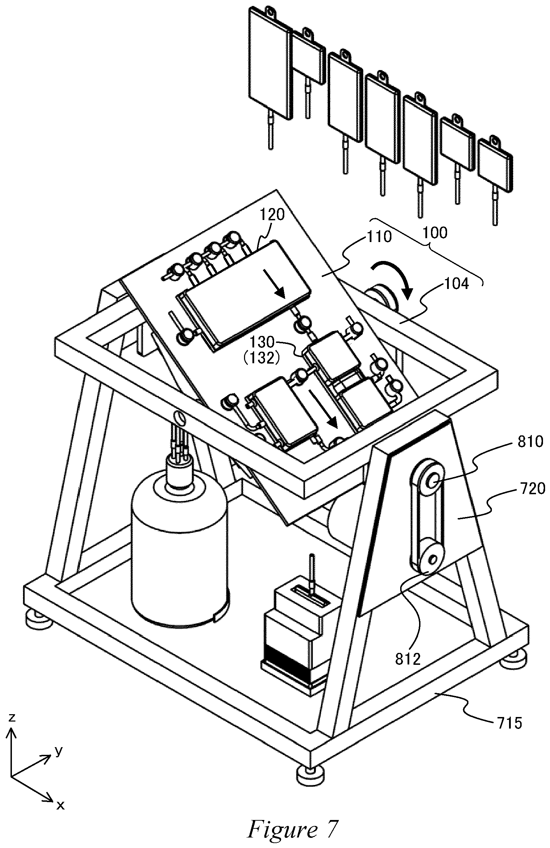

Following the washing step, the controller 200 performs a magnetic bead-separating step. In the magnetic bead-separating step, the controller 200 positions the treatment section 100 to be in the horizontal state and then elevates the magnet plate 155 to the adsorbing position where the magnet plate 155 is positioned in the vicinity of the bottom surface of the cell separating unit 120. At the adsorbing position, the magnetic beads are transferred towards and adsorbed by the plurality of magnets 158 shown in Figure 4, but the cells of interest separated from the magnetic beads are dispersed in the solution. While the magnetic beads are adsorbed by the magnet plate 155, the treating stage 110 is tilted at a predetermined angle as shown in Figure 7. The predetermined angle may be, for example, 45-90 degrees with respect to the horizontal state.

While the treating stage 110 is tilted, the valve of the tube connecting the cell separating unit 120 and the filter unit 130 is opened for a predetermined period of time so that the solution containing the cells of interest but not the magnetic beads is transferred to the filter unit 130 by the fall thereof via the tube. Once the valve of the tube connecting the cell separating unit 120 and the filter unit 130 is closed, the treating stage is moved to the horizontal state (Figure 2), thereby completing the magnetic bead-separating step.

Following the magnetic bead-separating step, the controller 200 performs a washing step for washing the extracted cells of interest. In the washing step, the controller 200 opens the valve of the tube connecting the second wash solution bag 350 and the first chamber 132 to transfer the wash solution in the second wash solution bag 350 to the first chamber 132 by the fall thereof via the tube. When the wash solution is transferred to the first chamber 132 by the fall thereof, the controller 200 also opens the valve of the tube connecting the second chamber 136 and the liquid waste tank 400. By doing so, the wash solution in the second wash solution bag 350 flows through the first chamber 132, the filter chamber 134 and the second chamber 136 in this order to be transferred to the liquid waste tank 400 by the fall thereof. When the wash solution flows through the filter section 134, the cells of interest held at the filter section 134 are washed, thereby removing unnecessary substances from the cells of interest.

After the completion of the washing step, the controller 200 closes the valve of the tube connecting the second chamber 136 and the liquid waste tank 400 and opens the valve of the tube connecting the first chamber 132 and the culture unit 140, thereby proceeding to the culture preparation step.

In the culture preparation step, the controller 200 opens the valve of the tube connecting the culture reagent solution bag 360 and the second chamber 136 and opens the valve of the tube connecting the culture solution bag 370 and the second chamber 136. By doing so, the culture reagent solution in the culture reagent solution bag 360 and the culture solution in the culture solution bag 370 are transferred to the second chamber 136 by the fall thereof.

The culture reagent solution that fell from the culture reagent solution bag 360 and the culture solution that fell from the culture solution bag 370 flow through the second chamber 136, the filter section 134, the first chamber 132 and the tube connecting the first chamber 132 and the culture unit 140 to be transferred to the culture unit 140. When the culture reagent solution and the culture solution flow through the filter section 134, the cells of interest trapped at the filter section 134 are transferred to the culture unit 140 together with the culture reagent solution and the culture solution. In order to promote separation of the cells of interest from the filter section 134, the bellows 380 may preferably be compressed to apply a pressure to the second chamber 136 to forcibly separate the cells of interest from the filter section 134. Moreover, in the culture preparation step, the treatment section 100 can preferably be tilted as shown in Figure 6 to promote the solution transfer. Following the culture preparation step, the valve of the tube connecting the culture unit 140 and the filter unit 130 is closed to proceed to the culture step. Here, instead of applying a pressure to the second chamber 136 by compressing the bellows 380, a pressure can be applied to the second chamber 136 by utilizing a pressure of CO₂ supplied from the CO₂ gas supply unit 600.

In the culture step, the controller 200 performs culture of the cells of interest which have been mixed with the culture reagent and the culture solution while maintaining a suitable temperature inside the culture unit 140 using the temperature regulating unit 160, and supplying CO₂ from the CO₂ gas supply unit 600. Furthermore, while performing the culture, the treating stage 100 and/or the entire treatment section 100 can be swung in the same motion as in the mixing step.

Following the culture step, the treatment section 100 is tilted to the state shown in Figure 6 and the valve of the tube connecting the culture unit 140 and the liquid waste tank 400 is opened, so that the solution containing the cultured highly active cells of interest is transferred to the storage bag 510 of the biological substance storage section 500 by the fall thereof. While the solution containing the cultured highly active cells of interest is kept in the storage bag 510, the storage bag 510 is cooled by the cooler 520.

### DESCRIPTION OF REFERENCE NUMERALS

- 1000: Sample treating device
- 100: Treatment section
- 104: Frame
- 110: Treating stage
- 120: Cell separating unit
- 130: Filter unit
- 140: Culture unit
- 200: Controller
- 300: Solution supply section
- 400: Liquid waste tank
- 500: Biological substance storage section
- 600: CO₂ gas supply unit
- 700: Housing
- 710: Window
- 720: Support
- 730: Support
- 810: First rotation shaft
- 820: Second rotation shaft
- 900: Magnet unit

## Claims

1. A sample treating device for treating a solution containing a sample to obtain a solution containing a biological substance of interest from the sample, the device comprising:
a treating stage for performing the treatment of the sample by mixing the solution containing the sample with a plurality of solutions;
a first rotating device for rotating the treating stage about a first rotation shaft;
a second rotating device for rotating the treating stage about a second rotation shaft which is different from the first rotation shaft; and
a controller for controlling rotations by the first rotating device and the second rotating device,
wherein the controller tilts the treating stage in multiple directions using the first rotating device and the second rotating device for carrying out the treatment, and the controller utilizes these tilts in multiple directions to transfer the solution containing the sample and/or the solution containing the biological substance to perform the treatment.

2. The sample treating device according to Claim 1, wherein the controller directs an operation of rotating the treating stage about the first rotation shaft and an operation of rotating the treating stage about the second rotation shaft for treating the sample.

3. The sample treating device according to either one of Claims 1 and 2, comprising:
a frame for rotatably holding the treating stage; and
a support for rotatably supporting the frame.

4. The sample treating device according to Claim 2, wherein the frame is rotatable in one piece with the first rotation shaft.

5. The sample treating device according to either one of Claims 3 and 4, wherein the first rotating device is provided on the support.

6. The sample treating device according to any one of Claims 1-5, wherein the treating stage is rotatable in one piece with the second rotation shaft.

7. The sample treating device according to Claim 6, wherein the second rotating device is provided on the frame.

8. The sample treating device according to any one of Claims 1-7, comprising a solution supply section which allows the solution containing the sample and the plurality of solutions to transfer to the treating stage by the fall thereof.

9. The sample treating device according to any one of Claims 1-8, wherein the treating stage is provided with a biological substance separating unit for separating the biological substance from the sample by using magnetic particles.

10. The sample treating device according to Claim 9, comprising a magnet plate for adsorbing the magnetic particles in the biological substance separating unit.

11. The sample treating device according to Claim 10, comprising a magnetic force controller for controlling the magnetic force of the magnet plate.

12. The sample treating device according to either one of Claims 10 and 11, wherein the controller tilts the treating stage using the first rotating device and/or the second rotating device while the biological substance having the magnetic particles attached thereto is adsorbed in the biological substance separating unit by the magnetic force of the magnet plate, thereby separating the biological substance having the magnetic particles attached thereto from the sample.

13. The sample treating device according to Claim 12, wherein the controller supplies a wash solution to the biological substance separating unit while the biological substance having the magnetic particles attached thereto is adsorbed in the biological substance separating unit, thereby separating the magnetic particles from the biological substance.

14. The sample treating device according to Claim 13, wherein the treating stage is provided with a filter unit for filtering the biological substance from the solution containing the biological substance separated from the magnetic particles.

15. The sample treating device according to Claim 14, comprising a solution pressurizer for applying a pressure to the solution passing through the filter unit to promote the filtration.

16. The sample treating device according to Claim 15, wherein the solution pressurizer is a bellows or a gas supply unit.

17. The sample treating device according to any one of Claims 14-16, wherein the controller tilts the treating stage so that the solution containing the biological substance is transferred from the biological substance separating unit to the filter unit by the fall thereof.

18. The sample treating device according to Claim 17, wherein the filter unit is provided with a first chamber, a filter section connected to the first chamber and a second chamber connected to the filter section, wherein the first chamber, the filter section and the second chamber are arranged along the first rotation shaft.

19. The sample treating device according to Claim 18, wherein the controller tilts the treating stage using the second rotating device so that the solution containing the biological substance is transferred to the first chamber by the fall thereof, the biological substance is filtered from the solution at the filter section, and the solution is disposed via the second chamber.

20. The sample treating device according to Claim 19, wherein the controller supplies a wash solution to the first chamber while the treating stage is positioned to be in the tilted state to wash the biological substance filtered at the filter section.

21. The sample treating device according to any one of Claims 14-20, wherein the treating stage is provided with a manipulation unit for manipulating the biological substance filtered by the filter unit.

22. The sample treating device according to Claim 21, wherein the filter unit and the manipulation unit are arranged along the second rotation shaft, and the controller tilts the treating stage using the first rotating device so that the solution containing the biological substance is transferred from the filter unit to the manipulation unit by the fall thereof.

23. The sample treating device according to Claim 22, wherein the biological substance is a cell, the manipulation unit is a culture unit for culturing the cell, and the stage is provided with a temperature regulating unit for regulating the temperature of the culture unit.

24. The sample treating device according to Claim 23, wherein, for culturing the cell, the controller directs an operation of swinging the treating stage about the first rotation shaft and/or directs an operation of swinging the treating stage about the second rotation shaft, thereby culturing the cells.

25. The sample treating device according to any one of Claims 1-24, comprising, beneath the treating stage, a biological substance storage section for storing the solution containing the biological substance.

26. The sample treating device according to Claim 25, wherein the biological substance storage section is provided with a cooler for cooling the solution containing the biological substance.

27. The sample treating device according to either one of Claims 25 and 26, wherein the biological substance storage section is provided with a measurement mechanism for measuring the biological substance.

28. A method for treating a sample by using the sample treating device according to any one of Claims 1-27, the method comprising the steps of:
tilting the treating stage in a first direction using the first rotating device; and
tilting the treating stage in a second direction using the second rotating device.
